# DEMANDE DE BREVET EUROPEEN

(11) **EP 2 008 646 A1**
(43) Date de publication de la demande: **31.12.2008**
(21) Numéro de dépôt: 08158194.4
(22) Date de dépôt: 13.06.2008
(51) Int. Cl.: A61K 8/85, A61Q 1/06

(54) **Composition cosmétique comprenant deux polyesters**

(30) Priorité: 21.06.2007 FR 0755935
(71) Demandeur: L'Oreal, 75008 Paris (FR)
(72) Inventeur: Barba, Claudia, 75009, PARIS (FR); Paute, Chrystel, 94100, SACLAY (FR); Ricard, Audrey, 75012, PARIS (FR); Giustiniani, Pascal, 92250, LA GARENNE COLOMBES (FR)
(74) Mandataire: Miszputen, Laurent

(57) **Abrégé**

La présente demande concerne une composition cosmétique comprenant :
- au moins un polyester susceptible d'être obtenu par réaction :
- d'au moins un polyol comprenant 3 à 6 groupes hydroxyles;
- d'au moins un acide monocarboxylique ramifié non aromatique;
- d'au moins un acide monocarboxylique aromatique, et
- d'au moins un acide polycarboxylique comprenant au moins 2 groupes carboxyliques COOH et/ou un anhydride cyclique d'un tel acide polycarboxylique,

- au moins un polyester susceptible d'être obtenu par réaction :
- d'au moins un polyol comprenant 3 à 6 groupes hydroxyles;
- d'au moins un acide monocarboxylique linéaire non aromatique;
- d'au moins un acide monocarboxylique aromatique, et
- d'au moins un acide polycarboxylique comprenant au moins 2 groupes carboxyliques COOH et/ou un anhydride cyclique d'un tel acide polycarboxylique

La demande concerne également un procédé de traitement cosmétique employant ladite composition, ainsi que l'utilisation de cette composition pour le soin ou le maquillage de la peau ou des lèvres.

## Description

La présente invention a trait à des compositions cosmétiques comprenant deux polymères polyesters différents, ainsi qu'à leur utilisation notamment dans les rouges à lèvres.

Les compositions selon l'invention peuvent être appliquées sur des supports comme la peau du visage ou du corps, les lèvres et les matières kératiniques telles que les cheveux, les cils, les sourcils et les ongles.

Il existe de nombreuses compositions cosmétiques pour lesquelles des propriétés de brillance du film déposé, après application sur les matières kératiniques (peau, lèvres, phanères), sont souhaitées. On peut citer par exemple les rouges à lèvres, les vernis à ongles ou encore certains produits capillaires.

Afin d'obtenir un tel résultat, il est possible d'associer des matières premières particulières, notamment des lanolines, avec des huiles dites brillantes, telles que les polybutènes qui présentent toutefois une viscosité élevée; ou des esters d'acide ou d'alcool gras dont le nombre de carbone est élevé; ou bien certaines huiles végétales; ou encore des esters résultants de l'estérification partielle ou totale d'un composé aliphatique hydroxylé avec un acide aromatique, comme décrit dans la demande de brevet EP1097699.

Il est également connu d'associer des lanolines avec des polyesters obtenus par réaction séquencée de l'huile de ricin avec l'acide isostéarique puis avec l'acide succinique, tel que décrit dans le brevet US6342527.

Pour améliorer la brillance du film déposé, ainsi que sa tenue, il a également été proposé d'utiliser des esters résultant de la condensation d'un polyol avec un acide carboxylique de type "néo", notamment dans FR2838049.

On peut également citer EP1457201, qui décrit une composition associant un polyester de triglycérides d'acides carboxyliques hydroxylés et une huile de faible masse moléculaire choisie parmi les polybutylènes, les polyisobutylènes hydrogénés, les polydécènes hydrogénés ou non, les copolymères de vinylpyrrolidones, les esters d'acides gras linéaires, les esters hydroxylés, les esters d'alcools gras ou d'acides gras ramifiés en C24-C28, les huiles siliconées et/ou les huiles d'origine végétale.

Dans la demande de brevet EP0792637, il est décrit une composition associant un ester aromatique et un polymère de type polybutène ou polyisobutène.

Dans la demande de brevet EP1155687, on décrit un procédé consistant à incorporer, dans une phase huileuse constituée d'une huile cosmétiquement acceptable, un organopolysiloxane possédant au moins 2 groupes susceptibles d'établir des liaisons hydrogènes.

Toutefois, ces compositions et associations, même si elles améliorent la brillance de manière significative, sont encore jugées insuffisantes dans une optique de longue tenue de cette brillance, dans le temps.

Les polymères utilisés dans le cadre de la présente invention sont de préférence des résines alkydes, lesquelles constituent une classe particulière de polyesters en étant le produit de réaction de polyols et d'acides polycarboxyliques, généralement modifié par des acides gras insaturés, tels que l'acide oléique, ou par des huiles insaturées, huile de soja ou de ricin par exemple.

Il a été décrit dans l'art antérieur des composition cosmétiques comprenant des polyesters. On peut en particulier citer le document FR2562793 qui décrit l'utilisation de benzoate de saccharose en association avec des résines toluène sulfonamide formaldéhyde; ou le document JP61246113 qui décrit l'utilisation de benzoate de sucrose en association avec une résine alkyde modifiée glycidyl versatate ester. On peut aussi citer WO2002243676 qui décrit l'utilisation d'une résine polyester néopentyl glycol trimellitate adipate en association avec des copolymères d'acrylates et de méthacrylates d'alkyle. On connaît encore JP58023614 qui décrit l'utilisation d'un polyester modifié obtenu par condensation du pentaérythritol avec de l'acide cis-4-cyclohexene-1,2-dicarboxylique et des acides gras d'huile de ricin puis réaction avec un composé dioxirane de type résine époxy; ou encore JP54011244 qui décrit l'utilisation d'un polyester modifié obtenu par condensation du dipentaérythritol avec de l'acide cyclohexane-1,2-dicarboxylique et des acides gras d'huile de ricin puis réaction avec un composé dioxirane de type résine époxy.

Les polyesters utilisés dans le cadre de la présente invention ont une structure différente des polyesters connus. En outre, lorsqu'ils sont formulés en association avec des ingrédients particuliers, ils permettent d'atteindre des propriétés cosmétiques égales voir supérieures aux performances déjà obtenues avec les polyesters connus.

Le but de la présente invention est de proposer des compositions cosmétiques dont la brillance et la tenue dans le temps de la brillance sont améliorées comparativement aux compositions de l'art antérieur contenant d'autres polyesters.

La demanderesse a découvert de façon surprenante et inattendue que deux polyesters particuliers conduisent à des compositions cosmétiques dont la tenue de la brillance est améliorée.

La présente invention a donc pour objet une composition cosmétique comprenant :
- d'au moins un polyester susceptible d'être obtenu par réaction :
   - d'au moins un polyol comprenant 3 à 6 groupes hydroxyles;
   - d'au moins un acide monocarboxylique ramifié non aromatique;
   - d'au moins un acide monocarboxylique aromatique, et
   - d'au moins un acide polycarboxylique comprenant au moins 2 groupes carboxyliques COOH et/ou un anhydride cyclique d'un tel acide polycarboxylique,
- d'au moins un polyester susceptible d'être obtenu par réaction :
   - d'au moins un polyol comprenant 3 à 6 groupes hydroxyles;
   - d'au moins un acide monocarboxylique linéaire non aromatique;
   - d'au moins un acide monocarboxylique aromatique, et
   - d'au moins un acide polycarboxylique comprenant au moins 2 groupes carboxyliques COOH et/ou un anhydride cyclique d'un tel acide polycarboxylique.

La composition de l'invention peut se présenter sous forme de pâte, de solide, de crème plus ou moins visqueuse. Elle peut être une émulsion huile-dans-eau ou eau-dans-huile, un gel anhydre rigide ou souple. En particulier, elle se présente sous forme coulée en stick ou en coupelle et plus spécialement sous forme d'un gel rigide anhydre notamment de stick anhydre.

Selon un autre de ses aspects, la présente invention a pour objet une composition cosmétique contenant :
- un premier polymère acide benzoïque / acide isophtalique / acide isostéarique / pentaérythritol, et
- un deuxième polymère acide benzoïque / acide isophtalique / acide stéarique / pentaérythritol.

Le ratio massique du premier et du deuxième polymère est avantageusement compris entre 50/1 et 2/1, par exemple entre 30/1 et 20/1.

Par "hydrocarboné", on entend un radical ou un composé formé essentiellement, voire constitué, d'atomes de carbone et d'hydrogène, et éventuellement d'atomes d'oxygène, d'azote, de soufre, de phosphore, et ne contenant pas d'atome de silicium ou de fluor. Elle peut contenir des groupes alcool, éther, acide carboxylique, amine et/ou amide. De préférence, le l'adjectif « hydrocarboné » désigne un radical ou un composé constitué uniquement d'atomes de carbone et d'hydrogène, et d'oxygène.

Par « ramifié », on entend un composé comprenant au moins une ramification. D'une façon plus générale, le nombre de ramifications d'une molécule correspond au nombre de groupes latéraux contenant au moins un atome de carbone et branchés sur la chaîne principale de la molécule, la chaîne principale correspondant à la plus longue chaîne carbonée de la molécule (voir Organic Chemistry, S.H. Pine, 5^{ème} Edition ; Mc Graw-Hill, chapitre 3).

### POLYESTERS (ou POLYCONDENSATS)

Les polyesters (appelés également par la suite polycondensats) sont avantageusement obtenus par réaction d'un polyol, d'un acide polycarboxylique, d'un acide monocarboxylique ramifié ou linéaire non aromatique, et d'un acide monocarboxylique aromatique.

Le premier polymère peut être obtenu par réaction d'un polyol, d'un acide polycarboxylique, d'un acide monocarboxylique ramifié non aromatique, et d'un acide monocarboxylique aromatique.

Le deuxième polymère est avantageusement obtenu par réaction d'un polyol, d'un acide polycarboxylique, d'un acide monocarboxylique linéaire non aromatique, et d'un acide monocarboxylique aromatique.

Selon un mode de réalisation, la teneur en acide monocarboxylique non aromatique est comprise entre 5 et 80% en poids, de préférence entre 20 et 70% en poids, par exemple de 25 à 65% en poids par rapport au poids total du polycondensat .

Selon un autre mode de réalisation, les polyesters sont avantageusement obtenus à partir de la réaction d'un polyol, d'un acide polycarboxylique et d'au moins un acide monocarboxylique non aromatique, ledit acide monocarboxylique étant dans une teneur importante.

Les polycondensats sont susceptibles d'être obtenus par estérification/polycondensation, selon les méthodes connues de l'homme du métier, des constituants décrits ci-après.

L'un des constituants nécessaires pour la préparation des polycondensats selon l'invention est un polyol, comprenant de préférence 3 à 6 groupes hydroxyles, notamment 3 à 4 groupes hydroxyles. On peut bien évidemment utiliser un mélange de tels polyols.

Ledit polyol peut notamment être un composé carboné, notamment hydrocarboné, linéaire, ramifié et/ou cyclique, saturé ou insaturé, comprenant 3 à 18 atomes de carbone, notamment 3 à 12, voire 4 à 10 atomes de carbone, et 3 à 6 groupes hydroxy (OH), et pouvant comprendre en outre un ou plusieurs atomes d'oxygène intercalés dans la chaîne (fonction éther).

Ledit polyol est de préférence un composé hydrocarboné saturé, linéaire ou ramifié, comprenant 3 à 18 atomes de carbone, notamment 3 à 12, voire 4 à 10 atomes de carbone, et 3 à 6 groupes hydroxy (OH).

Il peut être choisi parmi, seul ou en mélange :
- les triols, tels que le 1,2,4-butanetriol, le 1,2,6-hexanetriol, le triméthyloléthane, le triméthylolpropane, le glycérol;
- les tétraols, tels que le pentaérythritol (tétraméthylolméthane), l'érythritol, le diglycérol ou le ditriméthylolpropane;
- les pentols tels que le xylitol,
- les hexols tels que le sorbitol et le mannitol; ou encore le dipentaérythritol ou le triglycérol.

De préférence, le polyol est choisi parmi le glycérol, le pentaérythritol, le diglycérol, le sorbitol et leurs mélanges; et encore mieux le polyol est un tétraol comme le pentaérythritol.

Le polyol, ou le mélange de polyol, représente de préférence 10 à 30% en poids, notamment 12 à 25% en poids, et mieux 14 à 22% en poids, du poids total du polycondensat final.

Un autre constituant nécessaire pour la préparation du premier polyester selon l'invention est un acide monocarboxylique ramifié non aromatique. L'acide monocarboxylique ramifié non aromatique peut être saturé ou insaturé, comprenant 6 à 32 atomes de carbone, notamment 8 à 28 atomes de carbone et encore mieux 10 à 24, voire 12 à 20, atomes de carbone. On peut bien évidemment utiliser un mélange de tels acides monocarboxyliques non aromatiques.

Par acide monocarboxylique ramifié non aromatique, on entend un composé de formule RCOOH, dans laquelle R est un radical hydrocarboné saturé ou insaturé, ramifié, comprenant 5 à 31 atomes de carbone, notamment 7 à 27 atomes de carbone, et encore mieux 9 à 23 atomes de carbone, voire 11 à 19 atomes de carbone.

De préférence, le radical R est saturé. Encore mieux, ledit radical R est ramifié, en C5-C31, voire en C11-C21.

Dans un mode de réalisation particulier de l'invention, l'acide monocarboxylique ramifié non aromatique présente une température de fusion supérieure ou égale à 25°C, notamment supérieure ou égale à 28°C, voire à 30°C; on a en effet constaté que lorsque l'on emploie un tel acide, en particulier en quantité importante, il est possible, d'une part d'obtenir une bonne brillance et la tenue de ladite brillance, et d'autre part de réduire la quantité de cires usuellement présente dans la composition envisagée.

Parmi les acides monocarboxyliques non aromatiques ramifiés susceptibles d'être employés, on peut citer, seul ou en mélange, :
l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide isooctanoïque, l'acide isononanoïque, l'acide isostéarique.

De préférence, on peut utiliser l'acide 2-éthylhexanoïque, l'acide isooctanoïque, l'acide isoheptanoïque, l'acide isononanoïque, l'acide isostéarique, et leurs mélanges, et encore mieux l'acide isostéarique.

Ledit acide monocarboxylique ramifié non aromatique, ou le mélange desdits acides, représente de préférence 30 à 80% en poids, notamment 40 à 75% en poids, voire 45 à 70% en poids, et mieux 50 à 65% en poids, du poids total du polycondensat final.

Un autre constituant nécessaire pour la préparation du deuxième polyester selon l'invention est un acide monocarboxylique linéaire non aromatique. L'acide monocarboxylique non aromatique peut être saturé ou insaturé, comprenant 6 à 32 atomes de carbone, notamment 8 à 28 atomes de carbone et encore mieux 10 à 24, voire 12 à 20, atomes de carbone. On peut bien évidemment utiliser un mélange de tels acides monocarboxyliques non aromatiques.

Par acide monocarboxylique non aromatique, on entend un composé de formule RCOOH, dans laquelle R est un radical hydrocarboné saturé ou insaturé, linéaire, comprenant 5 à 31 atomes de carbone, notamment 7 à 27 atomes de carbone, et encore mieux 9 à 23 atomes de carbone, voire 11 à 19 atomes de carbone.

De préférence, le radical R est saturé. Encore mieux, ledit radical R est linéaire ou ramifié, et préférentiellement en C5-C31, voire en C11-C21.

Dans un mode de réalisation particulier de l'invention, l'acide monocarboxylique non aromatique présente une température de fusion supérieure ou égale à 25°C, notamment supérieure ou égale à 28°C, voire à 30°C; on a en effet constaté que lorsque l'on emploie un tel acide, en particulier en quantité importante, il est possible, d'une part d'obtenir une bonne brillance et la tenue de ladite brillance, et d'autre part de réduire la quantité de cires usuellement présente dans la composition envisagée.

Parmi les acides monocarboxyliques linéaires non aromatiques susceptibles d'être employés, on peut citer, seul ou en mélange, :
- les acides monocarboxyliques saturés tels que l'acide caproïque, l'acide caprylique, l'acide octanoïque, l'acide nonanoïque, l'acide décanoïque, l'acide laurique, l'acide tridécanoïque, l'acide myristique, l'acide palmitique, l'acide stéarique, l'acide arachidique, l'acide béhénique, l'acide cérotique (hexacosanoïque); l'acide cyclopentanecarboxylique, l'acide cyclopentaneacétique, l'acide 3-cyclopentyl-propionique, l'acide cyclohexanecarboxylique, l'acide cyclohexylacétique, l'acide 4-cyclohexylbutyrique;
- les acides monocarboxyliques insaturés mais non aromatiques, tels que l'acide caproléique, l'acide obtusilique, l'acide undécylénique, l'acide dodécylénique, l'acide lindérique, l'acide myristoléique, l'acide physétérique, l'acide tsuzuique, l'acide palmitoléique, l'acide oléique, l'acide pétrosélinique, l'acide vaccénique, l'acide élaidique, l'acide gondoïque, l'acide gadoléique, l'acide érucique, l'acide cétoléique, l'acide nervonique, l'acide linoléique, l'acide linolénique, l'acide arachidonique.

Parmi les acides monocarboxyliques linéaires non aromatiques cités précédemment ayant une température de fusion supérieure ou égale à 25°C, on peut citer, seul ou en mélange :
- parmi les acides monocarboxyliques saturés : l'acide décanoïque (caprique), l'acide laurique, l'acide tridécanoïque, l'acide myristique, l'acide palmitique, l'acide stéarique, l'acide arachidique, l'acide béhénique, l'acide cérotique (hexacosanoïque);
- parmi les acides monocarboxyliques insaturés mais non aromatiques : l'acide pétrosélinique, l'acide vaccénique, l'acide élaidique, l'acide gondoïque, l'acide gadoléique, l'acide érucique, l'acide nervonique.

De préférence, on peut utiliser l'acide laurique, l'acide myristique, l'acide nonanoïque, l'acide palmitique, l'acide stéarique, l'acide béhénique et leurs mélanges, et encore mieux l'acide stéarique seul.

Ledit acide monocarboxylique linéaire non aromatique, ou le mélange desdits acides, représente de préférence 30 à 80% en poids, notamment 40 à 75% en poids, voire 45 à 70% en poids, et mieux 50 à 65% en poids, du poids total du polycondensat final.

Un autre constituant nécessaire pour la préparation des polycondensats selon l'invention est un acide monocarboxylique aromatique. Cet acide peut comprendre 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone, notamment 2 à 12, voire 3 à 8 atomes de carbone.

On peut bien évidemment utiliser un mélange de tels acides monocarboxyliques aromatiques.

Par acide monocarboxylique aromatique, on entend un composé de formule R'COOH, dans laquelle R' est un radical hydrocarboné aromatique, comprenant 6 à 10 atomes de carbone, et en particulier les radicaux benzoïque et naphtoïque.

Ledit radical R' peut en outre être substitué par 1 à 3 radicaux alkyles, saturés ou insaturés, linéaires, ramifiés et/ou cycliques, comprenant 1 à 32 atomes de carbone, notamment 2 à 12, voire 3 à 8 atomes de carbone; et notamment choisis parmi méthyle, éthyle, propyle, isopropyle, butyle, isobutyle, terbutyle, pentyle, isopentyle, néopentyle, cyclopentyle, hexyle, cyclohexyle, heptyle, isoheptyle, octyle ou isooctyle.

Parmi les acides monocarboxyliques aromatiques susceptibles d'être employés, on peut citer, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl-benzoïque, l'acide 1-méthyl-2-naphtoïque, l'acide 2-isopropyl-1-naphtoïque.

De préférence, on peut utiliser l'acide benzoïque, l'acide 4-tert-butyl-benzoïque, l'acide o-toluique, l'acide m-toluique, l'acide 1-naphtoïque, seuls ou en mélanges; et encore mieux l'acide benzoïque seul.

Ledit acide monocarboxylique aromatique, ou le mélange desdits acides, représente de préférence 0,1 à 10% en poids, notamment 0,5 à 9,95% en poids, mieux encore de 1 à 9,5% en poids, voire 1,5 à 8% en poids, du poids total du polycondensat final.

Le polyester peut être obtenu à partir d'un acide monocarboxylique ramifié non aromatique, saturé ou insaturé, comprenant 10 à 32 atomes de carbone, notamment 12 à 28 atomes de carbone et encore mieux 12 à 24 atomes de carbone; et ayant une température de fusion supérieure ou égale à 25°C, notamment supérieure ou égale à 28°C, voire à 30°C. On peut bien évidemment utiliser un mélange de tels acides monocarboxyliques non aromatiques.

On a constaté que lorsque l'on emploie un tel acide, dans les quantités indiquées, il est possible, d'une part d'obtenir une bonne brillance et la tenue de ladite brillance, et d'autre part de réduire la quantité de cires usuellement présentes dans la composition envisagée. Par acide monocarboxylique ramifié non aromatique, on entend un composé de formule RCOOH, dans laquelle R est un radical hydrocarboné saturé ou insaturé, comprenant 9 à 31 atomes de carbone, notamment 11 à 27 atomes de carbone, et encore mieux 11 à 23 atomes de carbone.

De préférence, le radical R est saturé. Encore mieux, ledit radical R est linéaire ou ramifié, et préférentiellement en C11-C21.

Ledit acide monocarboxylique ramifié non aromatique de température de fusion supérieure ou égale à 25°C, ou le mélange desdits acides, représente de préférence 22 à 80% en poids, notamment 25 à 75% en poids, voire 27 à 70% en poids, et mieux 28 à 65% en poids, du poids total du polycondensat final.

Le polyester peut être obtenu à partir d'un acide monocarboxylique ramifié non aromatique, saturé ou insaturé, comprenant 6 à 32 atomes de carbone, notamment 8 à 28 atomes de carbone et encore mieux 10 à 20, voire 12 à 18, atomes de carbone; pouvant avoir une température de fusion strictement inférieure à 25°C, notamment inférieure à 20°C, voire à 15°C. On peut bien évidemment utiliser un mélange de tels acides monocarboxyliques non aromatiques.

Par acide monocarboxylique ramifié non aromatique, on entend un composé de formule RCOOH, dans laquelle R est un radical hydrocarboné saturé ou insaturé, linéaire, ramifié et/ou cyclique, comprenant 5 à 31 atomes de carbone, notamment 7 à 27 atomes de carbone, et encore mieux 9 à 19 atomes de carbone, voire 11 à 17 atomes de carbone.

De préférence, le radical R est saturé. Encore mieux, ledit radical R est linéaire ou ramifié, et préférentiellement en C5-C31.

Parmi les acides monocarboxyliques non aromatiques ayant une température de fusion inférieure à 25°C, susceptibles d'être employés, on peut citer, seul ou en mélange :
- parmi les acides monocarboxyliques saturés: l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide isooctanoïque, l'acide isononanoïque, l'acide isostéarique;

De préférence, on peut utiliser l'acide isooctanoïque, l'acide isononanoïque, l'acide isostéarique, et leurs mélanges, et encore mieux l'acide isostéarique seul.

Ledit acide monocarboxylique ramifié non aromatique de température de fusion inférieure à 25°C, ou le mélange desdits acides, représente de préférence 0,1 à 35% en poids, notamment 0,5 à 32% en poids, voire 1 à 30% en poids, et mieux 2 à 28% en poids, du poids total du polycondensat final.

Un autre constituant nécessaire pour la préparation des polycondensats selon l'invention est un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique. On peut bien évidemment utiliser un mélange de tels acides polycarboxyliques et/ou d'anhydrides.

Ledit acide polycarboxylique peut notamment être choisi parmi les acides polycarboxyliques linéaires, ramifiés et/ou cycliques, saturés ou insaturés, voire aromatiques, comprenant 3 à 50, notamment 3 à 40, atomes de carbone, en particulier 3 à 36, voire 3 à 18, et encore mieux 4 à 12 atomes de carbone, voire 4 à 10 atomes de carbone;

Ledit acide comprend au moins deux groupes carboxyliques COOH, de préférence de 2 à 4 groupes COOH.

De préférence, ledit acide polycarboxylique est aliphatique et comprend 3 à 36 atomes de carbone, notamment 3 à 18 atomes de carbone, voire 4 à 12 atomes de carbone; ou bien ledit acide polycarboxylique est aromatique et comprend 8 à 12 atomes de carbone. Il comprend de préférence 2 à 4 groupes COOH.

L'anhydride cyclique d'un tel acide polycarboxylique peut notamment répondre à l'une des formules suivantes : 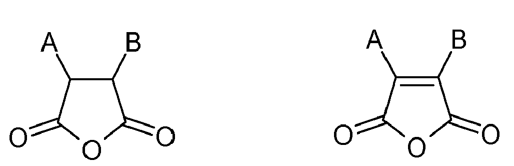 dans lesquelles les groupements A et B sont, indépendamment l'un de l'autre, :
- un atome d'hydrogène,
- un radical carboné, aliphatique, saturé ou insaturé, linéaire, ramifié et/ou cyclique, ou bien aromatique; comprenant 1 à 16 atomes de carbone, notamment 2 à 10 atomes de carbone, voire 4 à 8 atomes de carbone, notamment méthyle ou éthyle;
- ou bien A et B pris ensemble forment un cycle comprenant au total 5 à 7, notamment 6 atomes de carbone, saturé ou insaturé, voire aromatique.

De préférence, A et B représentent un atome d'hydrogène ou forment ensemble un cycle aromatique comprenant au total 6 atomes de carbone.

Parmi les acides polycarboxyliques ou leurs anhydrides, susceptibles d'être employés, on peut citer, seul ou en mélange :
- les acides dicarboxyliques tels que l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide tétrahydrophtalique, l'acide hexahydrophtalique, l'acide pimélique, l'acide sébacique, l'acide azélaïque, l'acide glutarique, l'acide adipique, l'acide fumarique, l'acide maléïque, l'acide itaconique, les dimères d'acides gras (notamment en C36) tels que les produits commercialisés sous les dénominations Pripol 1006, 1009, 1013 et 1017, par Uniqema;
- les acides tricarboxyliques tels que l'acide cyclohexanetricarboxylique, l'acide trimellitique, l'acide 1,2,3-benzènetricarboxylique, l'acide 1,3,5-benzènetricarboxy-lique;
- les acides tétracarboxyliques tels que l'acide butanetétracarboxylique et l'acide pyroméllitique,
- les anhydrides cycliques de ces acides et notamment l'anhydride phtalique, l'anhydride trimellitique, l'anhydride maléïque et l'anhydride succinique.

De préférence, on peut utiliser l'acide adipique, l'anhydride phtalique et/ou l'acide isophtalique, et encore mieux l'acide isophtalique seul.

Ledit acide polycarboxylique et/ou son anhydride cyclique, représente de préférence 5 à 40% en poids, notamment 10 à 30% en poids, et mieux 14 à 25% en poids, du poids total du polycondensat final.

Le polycondensat peut en outre comprendre une silicone à fonction hydroxyle (OH) et/ou carboxylique (COOH).

Elle peut comprendre 1 à 3 fonctions hydroxyle et/ou carboxylique, et comprend de préférence deux fonctions hydroxyle ou bien deux fonctions carboxyliques.

Ces fonctions peuvent être situées en bout de chaîne ou dans la chaîne, mais avantageusement en bout de chaîne.

On emploie de préférence des silicones ayant une masse moléculaire moyenne en poids (Mw) comprise entre 300 et 20000, notamment 400 et 10 000, voire 800 et 4000.

Cette silicone peut être de formule : 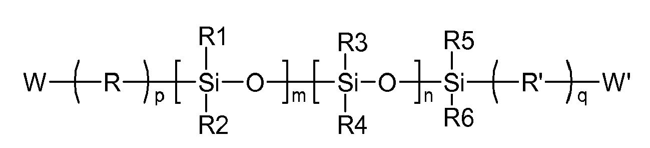 dans laquelle :
- W et W' sont, indépendamment l'un de l'autre, OH ou COOH; de préférence W=W';
- p et q sont, indépendamment l'un de l'autre, égaux à 0 ou 1,
- R et R' sont, indépendamment l'un de l'autre, un radical divalent carboné, notamment hydrocarboné, saturé ou insaturé, voire aromatique, linéaire, ramifié et/ou cyclique; comprenant 1 à 12 atomes de carbone, notamment 2 à 8 atomes de carbone, et comprenant éventuellement en outre 1 ou plusieurs hétéroatomes choisis parmi O, S et N, notamment O (éther);
   notamment R et/ou R' peuvent être de formule -(CH₂)ₐ- avec a=1-12, et notamment méthylène, éthylène, propylène, phénylène;
   ou bien de formule -[(CH₂)ₓO]_{z}- avec x = 1, 2 ou 3 et z = 1-10; en particulier x=2 ou 3 et z=1-4; et mieux x=3 et z=1.
- R1 à R6 sont, indépendamment l'un de l'autre, un radical carboné linéaire, ramifié et/ou cyclique, saturé ou insaturé voire aromatique; comprenant 1 à 20 atomes de carbone, notamment 2 à 12 atomes de carbone; de préférence, R1 à R6 sont saturés ou bien aromatiques, et peuvent notamment être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle.
- m et n sont, indépendamment l'un de l'autre, des entiers compris entre 1 et 140, et sont tels que la masse moléculaire moyenne en poids (Mw) de la silicone est comprise entre 300 et 20 000, notamment entre 400 et 10 000, voire entre 800 et 4000.

On peut notamment citer les polyalkylsiloxanes α,ω-diol ou α,ω-dicarboxylique, et notamment les polydiméthysiloxanes α,ω-diol et les polydiméthylsiloxanes α,ω-dicarboxylique; les polyarylsiloxanes α,ω-diol ou α,ω-dicarboxylique et notamment les polyphénylsiloxanes α,ω-diol ou α,ω-dicarboxylique; les polyarylsiloxanes à fonctions silanol tels que le polyphénylsiloxane; les polyalkylsiloxanes à fonctions silanol tels que le polydiméthylsiloxane; les polyaryl/alkylsiloxanes à fonctions silanol tels que le polyphényl/méthylsiloxane ou encore le polyphényl/propylsiloxane.

Tout particulièrement, on utilisera les polydiméthysiloxanes α,ω-diol de masse moléculaire moyenne en poids (Mw) comprise entre 400 et 10 000, voire entre 500 et 5000, et notamment entre 800 et 4000.

Lorsqu'elle est présente, ladite silicone peut de préférence représenter 0,1 à 15% en poids, notamment 1 à 10% en poids, voire 2 à 8% en poids, du poids du polycondensat.

Selon un mode de réalisation, le premier polyester est susceptible d'être obtenu par réaction :
- d'au moins un polyol comprenant 3 à 6 groupes hydroxyles;
- d'au moins un acide monocarboxylique ramifié non aromatique comprenant 6 à 32 atomes de carbone;
- d'au moins un acide monocarboxylique aromatique comprenant 7 à 11 atomes de carbone,
- d'au moins un acide polycarboxylique comprenant au moins 2 groupes carboxyliques COOH et/ou un anhydride cyclique d'un tel acide polycarboxylique

Selon un mode de réalisation, le deuxième polyester est susceptible d'être obtenu par réaction :
- d'au moins un polyol comprenant 3 à 6 groupes hydroxyles;
- d'au moins un acide monocarboxylique linéaire non aromatique comprenant 6 à 32 atomes de carbone;
- d'au moins un acide monocarboxylique aromatique comprenant 7 à 11 atomes de carbone,
- d'au moins un acide polycarboxylique comprenant au moins 2 groupes carboxyliques COOH et/ou un anhydride cyclique d'un tel acide polycarboxylique

De préférence, l'acide monocarboxylique non aromatique ne comprend pas de groupe OH libre.

Selon un mode de réalisation, le polycondensat peut être obtenu par réaction :
- de 10 à 30% en poids, par rapport au poids total du polycondensat, d'au moins un polyol comprenant 3 à 6 groupes hydroxyles;
- de 30 à 80% en poids, par rapport au poids total du polycondensat, d'au moins un acide monocarboxylique non aromatique, saturé ou insaturé, (linéaire ou ramifié selon le polyester que l'on souhaite préparer) comprenant 6 à 32 atomes de carbone;
- de 0,1 à 10 % en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
- de 5 à 40% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique.

Selon un mode de réalisation, ledit polycondensat est susceptible d'être obtenu par réaction :
- de 15 à 30% en poids, par rapport au poids total du polycondensat, d'au moins un polyol comprenant 3 à 6 groupes hydroxyles;
- de 5 à 40% en poids, par rapport au poids total du polycondensat, d'au moins un acide monocarboxylique non aromatique, saturé ou insaturé, (linéaire ou ramifié selon le polyester que l'on souhaite préparer), comprenant 6 à 32 atomes de carbone;
- de 10 à 55 % en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
- de 10 à 25% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique.

De préférence, le premier polyester est susceptible d'être obtenu par réaction :
- d'au moins un polyol choisi parmi, seul ou en mélange, le 1,2,6-hexanetriol, le triméthyloléthane, le triméthylolpropane, le glycérol; le pentaérythritol, l'érythritol, le diglycérol, le ditriméthylolpropane; le xylitol, le sorbitol, le mannitol, le dipentaérythritol et/ou le triglycérol;
   présent de préférence en une quantité de 10 à 30% en poids, notamment 12 à 25% en poids, et mieux 14 à 22% en poids, par rapport au poids total du polycondensat final;
- d'au moins un acide monocarboxylique ramifié non aromatique choisi parmi, seul ou en mélange, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide isooctanoïque, l'acide isononanoïque, l'acide isostéarique;
   présent de préférence en une quantité de 30 à 80% en poids, notamment 40 à 75% en poids, et mieux 45 à 70% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque;
   présent de préférence en une quantité de 0,1 à 10% en poids, notamment 1 à 9,5% en poids, voire 1,5 à 8% en poids, par rapport au poids total du polycondensat final; et
- d'au moins un acide polycarboxylique ou un de ses anhydrides, choisi parmi, seul ou en mélange, l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque; l'acide cyclohexanetricarboxylique, l'acide trimellitique, l'acide 1,2,3-benzènetricarboxylique, l'acide 1,3,5-benzènetricarboxylique; l'acide butanetétracarboxylique, l'acide pyroméllitique, l'anhydride phtalique, l'anhydride trimellitique, l'anhydride maléïque et l'anhydride succinique;
   présent de préférence en une quantité de 5 à 40% en poids, notamment 10 à 30% en poids, et mieux 14 à 25% en poids, par rapport au poids total du polycondensat final.

De préférence, le deuxième polyester est susceptible d'être obtenu par réaction :
- d'au moins un polyol choisi parmi, seul ou en mélange, le 1,2,6-hexanetriol, le triméthyloléthane, le triméthylolpropane, le glycérol; le pentaérythritol, l'érythritol, le diglycérol, le ditriméthylolpropane; le xylitol, le sorbitol, le mannitol, le dipentaérythritol et/ou le triglycérol;
   présent de préférence en une quantité de 10 à 30% en poids, notamment 12 à 25% en poids, et mieux 14 à 22% en poids, par rapport au poids total du polycondensat final;
- d'au moins un acide monocarboxylique linéaire non aromatique choisi parmi, seul ou en mélange, l'acide caproïque, l'acide caprylique, l'acide octanoïque, l'acide nonanoïque, l'acide décanoïque, l'acide laurique, l'acide tridécanoïque, l'acide myristique, l'acide palmitique, l'acide stéarique, l'acide arachidique, l'acide béhénique, l'acide cérotique (hexacosanoïque);
   présent de préférence en une quantité de 30 à 80% en poids, notamment 40 à 75% en poids, et mieux 45 à 70% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque;
   présent de préférence en une quantité de 0,1 à 10% en poids, notamment 1 à 9,5% en poids, voire 1,5 à 8% en poids, par rapport au poids total du polycondensat final; et
- d'au moins un acide polycarboxylique ou un de ses anhydrides, choisi parmi, seul ou en mélange, l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque; l'acide cyclohexanetricarboxylique, l'acide trimellitique, l'acide 1,2,3-benzènetricarboxylique, l'acide 1,3,5-benzènetricarboxylique; l'acide butanetétracarboxylique, l'acide pyroméllitique, l'anhydride phtalique, l'anhydride trimellitique, l'anhydride maléïque et l'anhydride succinique;
   présent de préférence en une quantité de 5 à 40% en poids, notamment 10 à 30% en poids, et mieux 14 à 25% en poids, par rapport au poids total du polycondensat final.

Selon un autre mode de réalisation, le premier et le deuxième polycondensat sont tous deux susceptibles d'être obtenu par réaction :
- de 10 à 30% en poids, par rapport au poids total du polycondensat, d'au moins un polyol comprenant 3 à 6 groupes hydroxyles;
- de 22 à 80% en poids, par rapport au poids total du polycondensat, d'au moins un acide monocarboxylique ramifié non aromatique, saturé ou insaturé, comprenant 10 à 32 atomes de carbone, et ayant une température de fusion supérieure ou égale à 25°C;
- de 0,1 à 35% en poids, par rapport au poids total du polycondensat, d'au moins un acide monocarboxylique linéaire non aromatique, saturé ou insaturé, comprenant 6 à 32 atomes de carbone, et ayant une température de fusion strictement inférieure à 25°C;
- de 0,1 à 10 % en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
- de 5 à 40% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique.

Préférentiellement, le premier polycondensat est susceptible d'être obtenu par réaction :
- d'au moins un polyol choisi parmi, seul ou en mélange, le glycérol, le pentaérythritol, le sorbitol et leurs mélanges, et encore mieux le pentaérythritol seul; présent en une quantité de 10 à 30% en poids, notamment 12 à 25% en poids, et mieux 14 à 22% en poids, par rapport au poids total du polycondensat final;
- d'au moins un acide monocarboxylique ramifié non aromatique choisi parmi, seul ou en mélange, l'acide 2-éthylhexanoïque, l'acide isooctanoïque, l'acide laurique, l'acide palmitique, l'acide isostéarique, l'acide isononanoïque, l'acide stéarique, l'acide béhénique et leurs mélanges, et encore mieux l'acide isostéarique seul ou l'acide stéarique seul;
   présent en une quantité de 30 à 80% en poids, notamment 40 à 75% en poids, et mieux 45 à 70% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide 1-naphtoïque, et encore mieux l'acide benzoïque seul; présent en une quantité de 0,1 à 10% en poids, notamment 1 à 9,5% en poids, voire 1,5 à 8% en poids par rapport au poids total du polycondensat final; et
- d'au moins un acide polycarboxylique ou un de ses anhydrides, choisi parmi, seul ou en mélange, l'anhydride phtalique et l'acide isophtalique, et encore mieux l'acide isophtalique seul; présent en une quantité de 5 à 40% en poids, notamment 10 à 30% en poids, et mieux 14 à 25% en poids, par rapport au poids total du polycondensat final.

De préférence, le premier et/ou le deuxième polycondensat présente :
- un indice d'acide, exprimé en mg d'hydroxyde de potassium par g de polycondensat, supérieur ou égal à 1; notamment compris entre 2 et 30, et encore mieux compris entre 2,5 et 15; et/ou
- un indice d'hydroxyle exprimé en mg d'hydroxyde de potassium par g de polycondensat, supérieur ou égal à 40; notamment compris entre 40 et 120, et encore mieux compris entre 45 et 80.

Ces indices d'acide et d'hydroxyle peuvent être aisément déterminés par l'homme du métier par les méthodes analytiques habituelles.

De préférence, le premier et/ou le deuxième polycondensat présente une masse moléculaire moyenne en poids (Mw) comprise entre 1500 et 300 000, voire entre 2000 et 200 000, et notamment entre 3000 et 100 000.

Le poids moléculaire moyen peut être déterminé par chromatographie par perméation sur gel ou par diffusion de la lumière, selon la solubilité du polymère considéré.

De préférence, le premier et/ou le deuxième polycondensat présente une viscosité, mesurée à 110°C, comprise entre 20 et 4000 mPa.s, notamment entre 30 et 3500 mPa.s, voire entre 40 et 3000 mPa.s et encore mieux entre 50 et 2500 mPa.s. Cette viscosité est mesurée de la manière décrite avant les exemples.

Le premier et/ou le deuxième polycondensat peut être préparé par les procédés d'estérification/polycondensation usuellement employés par l'homme du métier. A titre d'illustration, un procédé général de préparation consiste :
- à mélanger le polyol et les acides monocarboxyliques aromatiques et non aromatiques,
- à chauffer le mélange sous atmosphère inerte, d'abord jusqu'à la température de fusion (généralement 100-130°C) et ensuite à une température comprise entre 150 et 220°C jusqu'à consommation complète des acides monocarboxyliques (atteint lorsque l'indice d'acide est inférieur ou égal à 1), de préférence en distillant au fur et à mesure l'eau formée, puis
- à éventuellement refroidir le mélange à une température comprise entre 90 et 150°C,
- à ajouter l'acide polycarboxylique et/ou l'anhydride cyclique, et optionnellement la silicone à fonctions hydroxyles ou carboxyliques, en une seule fois ou de façon séquencée, puis
- à chauffer à nouveau à une température inférieure ou égale à 220°C, notamment comprise entre 170 et 220°C, de préférence en continuant à éliminer l'eau formée, jusqu'à l'obtention des caractéristiques requises en terme d'indice d'acide, de viscosité, d'indice d'hydroxyle et de solubilité.

Il est possible d'ajouter des catalyseurs d'estérification conventionnels, par exemple de type acide sulfonique (notamment à une concentration pondérale comprise entre 1 et 10%) ou type titanate (notamment à une concentration pondérale comprise entre 5 et 100 ppm).

Il est également possible de réaliser la réaction, en tout ou en partie, dans un solvant inerte tel que le xylène et/ou sous une pression réduite, pour faciliter l'élimination de l'eau. Avantageusement, on n'utilise ni catalyseur ni solvant.

Ledit procédé de préparation peut comprendre en outre une étape d'addition d'au moins un agent antioxydant dans le milieu réactionnel, notamment à une concentration pondérale comprise entre 0,01 et 1 %, par rapport au poids total de monomères, de façon à limiter les éventuelles dégradations liées à un chauffage prolongé.

L'agent antioxydant peut être de type primaire ou de type secondaire, et peut être choisi parmi les phénols encombrés, les amines secondaires aromatiques, les composés organophosphorés, les composés soufrés, les lactones, les bisphénols acrylés; et leurs mélanges.

Parmi les antioxydants particulièrement préférés, on peut notamment citer le BHT, le BHA, le TBHQ, le 1,3,5-trimethyl-2,4,6,tris(3,5-di-tertbutyl-4-hydroxybenzyl)-benzène, l'octadecyl-3,5,di-tertbutyl-4-hydroxycinnamate, le tetrakis-methylene-3-(3,5-di-tertbutyl-4-hydroxy-phenyl)propionate méthane, l'octadecyl-3-(3,5-di-tertbutyl-4-hydroxyphenyl)propionate 2,5-di-tertbutyl hydroquinone, le 2,2-methyl-bis-(4-methyl-6-tertbutyl phénol), le 2,2-methylene-bis-(4-ethyl-6-tertbutyl phénol), le 4,4-butylidene-bis(6-tertbutyl-m-cresol), le N,N-hexamethylene bis(3,5-di-tertbutyl-4-hydroxyhydrocinnamamide), le pentaerythritol tetrakis (3-(3,5-di-tertbutyl-4-hydroxyphenyl)propionate) notamment celui commercialisé par CIBA sous le nom IRGANOX 1010; l'octadecyl 3-(3,5-di-tertbutyl-4-hydroxphenyl) propionate notamment celui commercialisé par CIBA sous le nom IRGANOX 1076; la 1,3,5-tris(3,5-di-tertbutyl-4-hydroxybenzyl)-1,3,5-triazine-2,4,6(1H,3H,5H)trione notamment celui commercialisée par Mayzo of Norcross, Ga sous le nom BNX 3114; le di(stearyl)pentaerythritol diphosphite, le tris(2,4-ditertbutyl phenyl)phosphite notamment celui commercialisé par CIBA sous le nom IRGAFOS 168; le dilauryl thiodipropionate notamment celui commercialisé par CIBA sous le nom IRGANOX PS800; le bis(2,4-di-tertbutyl)pentaerythritol diphosphite notamment celui commercialisé par CIBA sous le nom IRGAFOS 126; le bis(2,4-bis)[2-phénylpropan-2-yl]phényl)pentaérythritol diphosphite, le triphénylphosphite, le (2,4-di-tertbutylphenyl)pentaerythritol diphosphite notamment celui commercialisé par GE Specialty Chemicals sous le nom ULTRANOX 626; le tris(nonylphenyl)phosphite notamment celui commercialisé par CIBA sous le nom IRGAFOS TNPP; le mélange 1:1 de N,N-hexamethylenebis(3,5-di-tertbutyl-4-hydroxy-hydrocinnamamide) et de tris(2,4-ditertbutylphenyl)phosphate notamment celui commercialisé par CIBA sous le nom Irganox B 1171; le tétrakis (2,4-di-tert-butylphényl)phosphite notamment celui commercialisé par CIBA sous le nom IRGAFOS P-EPQ; le distéarylthiodipropionate notamment celui commercialisé par CIBA sous le nom IRGANOX PS802; le 2,4-bis(octylthiométhyl)o-crésol notamment celui commercialisé par CIBA sous le nom IRGANOX 1520; le 4,6-bis(dodécylthiométhyl)o-crésol notamment celui commercialisé par CIBA sous le nom IRGANOX 1726.

Le premier polyester peut être avantageusement présent en une quantité totale comprise entre 1 et 50% en poids, notamment entre 10 et 45% en poids, voire entre 10 et 20% en poids par rapport au poids de la composition.

Le deuxième polyester peut être avantageusement présent en une quantité totale comprise entre 0,1 et 20% en poids, notamment entre 0,2 et 10% en poids, voire entre 0,5 et 2% en poids par rapport au poids de la composition

La quantité totale de polyesters présente dans les compositions dépend bien entendu du type de composition et des propriétés recherchées et peut varier à l'intérieur d'une gamme très large, comprise généralement entre 0,1 et 70% en poids, de préférence entre 1 et 50% en poids, notamment entre 10 et 45% en poids, voire entre 20 et 40% en poids, et mieux entre 25 et 35% en poids, par rapport au poids de la composition cosmétique.

Selon un mode de mise en oeuvre, la quantité totale de polycondensats est comprise entre 10 et 20 % en poids.

### HUILE NON VOLATILE

La composition selon l'invention comprend avantageusement une huile non volatile. L'huile non volatile peut représenter 1 à 90% en poids de la composition, notamment de 5 à 75% en poids, en particulier de 10 à 60% en poids, voire de 25 à 55% en poids, du poids total de la composition.

Selon un mode de mise en oeuvre, l'huile non volatile peut représenter de 35 à 60 % en poids.

Au sens de la présente invention, on entend par "huile non-volatile", une huile ayant une pression de vapeur inférieure à 0,13 Pa. Les huiles non volatiles peuvent être des huiles hydrocarbonées, des huiles siliconées, des huiles fluorées, ou leurs mélanges.

Au sens de la présente invention, on entend par "huile siliconée", une huile comprenant au moins un atome de silicium, et notamment au moins un groupe Si-O.

On entend par "huile hydrocarbonée", une huile contenant principalement des atomes d'hydrogène et de carbone et éventuellement des atomes d'oxygène, d'azote, de soufre et/ou de phosphore.

On entend par « hydrocarbure » une huile ne contenant que des atomes d'hydrogène et de carbone.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine végétale telles que les esters de phytostéaryle, tels que l'oléate de phytostéaryle, l'isostéarate de phytostéaryle et le glutamate de lauroyl/octyldodécyle/phytostéaryle (AJINOMOTO, ELDEW PS203), les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STEARINERIES DUBOIS ou ceux vendus sous les dénominations MIGLYOL 810^{®}, 812^{®} et 818^{®} par la société DYNAMIT NOBEL,
- les éthers de synthèse ayant de 10 à 40 atomes de carbone;
- les hydrocarbures linéaires ou ramifiés, d'origine minérale ou synthétique tels que la vaseline, les polydécènes, le polyisobutène hydrogéné tel que le Parleam ®, le squalane et leurs mélanges, et en particulier le polyisobutène hydrogéné,
- les esters de synthèse comme les huiles de formule R₁COOR₂ dans laquelle R₁ représente le reste d'un acide linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10.

Les esters peuvent être notamment choisis parmi les esters, notamment d'acide gras comme par exemple l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en C₁₂ à C₁₅, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle ;
- les esters de polyols et les esters du pentaérythritol, comme le tétrahydroxystéarate/tétraisostéarate de dipentaérythritol,
- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges, et
- les carbonates de di-alkyle, les 2 chaînes alkyles pouvant être identiques ou différentes, tel que le dicaprylyl carbonate commercialisé sous la dénomination CETIOL CC^{®}, par COGNIS.

Les huiles de silicone non volatiles utilisables dans la composition peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicones de viscosité inférieure ou égale à 100 cSt, et leurs mélanges.

Selon un autre mode de mise en oeuvre, l'huile siliconée répond à la formule 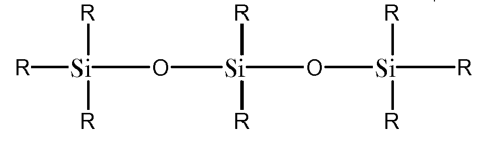 dans laquelle les groupements R représentent indépendamment les uns des autres un méthyle ou un phényle. De préférence dans cette formule, ledit organopolysiloxane comprend au moins trois groupes phenyls, par exemple au moins quatre ou au moins cinq. Des mélanges des organopolysiloxanes phénylés décrits précédemment peuvent être utilisés.

On peut citer par exemples des mélanges d'organopolysiloxane triphénylé, tétra- ou penta-phénylé.

Selon un autre mode de mise en oeuvre, l'huile siliconée répond à la formule 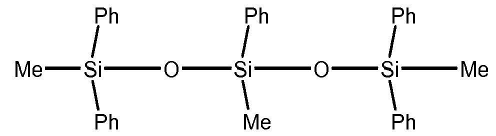 dans laquelle Me représente méthyle, Ph représente phényle. Une telle silicone phénylée est notamment fabriquée par Dow Corning sous la reference Dow Corning 555 Cosmetic Fluid (nom INCI : trimethyl pentaphenyl trisiloxane). La référence Dow Corning 554 Cosmetic Fluid peut aussi être utilisée.

L'huile non volatile est de préférence apolaire, au sens où son paramètre de solubilité delta a est égal à 0.

### CIRE

La composition peut contenir une cire. Par cire au sens de la présente invention, on entend un composé lipophile, solide à température ambiante (25°C), à changement d'état solide/liquide réversible, ayant un point de fusion supérieur ou égal à 30°C pouvant aller jusqu'à 120°C.

Le point de fusion de la cire peut être mesuré à l'aide d'un calorimètre à balayage différentiel (D.S.C.), par exemple le calorimètre vendu sous la dénomination DSC 30 par la Société METTLER.

Les cires peuvent être hydrocarbonées, fluorées et/ou siliconées. En particulier, les cires présentent une température de fusion supérieure à 25°C et mieux supérieure à 45°C. Comme cire utilisable dans la première, on peut citer des cires hydrocarbonées linéaires. Leur point de fusion est avantageusement supérieur à 35°C, par exemple supérieur à 55°C, de préférence supérieur à 80°C.

Les cires hydrocarbonées linéaires sont avantageusement choisies parmi les alcanes linéaires substitués, les alcanes linéaires non substitués, les alcènes linéaires non substitués, les alcènes linéaires substitués, un composé non substitué étant uniquement composé de carbone et d'hydrogène. Les substituants mentionnés précédemment ne contenant pas d'atomes de carbone.

Les cires hydrocarbonées linéaires incluent les polymères et copolymères de l'éthylène, de poids moléculaire compris entre 400 et 800, par exemple la Polywax 500 ou Polywax 400 commercialisée par New Phase Technologies.

Les cires hydrocarbonées linéaires incluent les cires de paraffine linéaires, comme les paraffines S&P 206, S&P 173 et S&P 434 de Strahl & Pitsch.

Les cires hydrocarbonées linéaires incluent les alcools linéaires à longue chaîne, comme les produits comprenant un mélange de polyéthylène et d'alcools en comprenant 20 à 50 atomes de carbones, notamment le Performacol 425 ou le Performacol 550 (mélange dans les proportions 20/80) commercialisés par New Phase Technologies.

Des exemples de cires siliconées sont par exemple
- Les C20-24 alkyl méthicone, C24-28 alkyl diméthicone, C20-24 alkyl diméthicone, C24-28 alkyl diméthicone commercialisées par Archimica Fine Chemicals sous la référence SilCare 41 M40, SilCare 41 M50, SilCare 41 M70 and SilCare 41 M80,
- Les stéaryl diméthicone de référence SilCare 41 M65 commercialisées par Archimica ou de référence DC-2503 commercialisée par Dow-Corning
- Les stéaroxytriméthylsilane vendues sous la référence SilCare 1 M71 ou DC-580
- Les produits ABIL Wax 9810, 9800, or 2440 de Wacker-Chemie GmbH,

Les C30-45 alkyl méthicone commercialisées par Dow Corning sous la référence AMS-C30 Wax, de même que les C30-45 alkyl diméthicone commercialisées sous la référence SF1642 ou SF-1632 par General Electric.

La quantité de cire dans la composition selon l'invention peut aller de 5 à 70% en poids, par rapport au poids total de la composition, de préférence de 5 à 40% en poids, et mieux de 10 à 30% en poids.

### MATIERE COLORANTE

La composition selon l'invention peut contenir une matière colorante, à raison de 0,5 à 50% de matière colorante, de préférence de 2 à 40 % et mieux de 5 à 30%, par rapport au poids total de la composition.

La matière colorante peut être tout composé minéral et/ou organique, présentant une absorption entre 350 et 700 nm, ou capable de générer un effet optique comme la réflexion de la lumière incidente ou des interférences par exemple.

Les matières colorantes utiles dans la présente invention sont choisies parmi tous les pigments organiques et / ou minéraux connus de la technique, notamment ceux qui sont décrits dans l'encyclopédie de technologie chimique de Kirk-Othmer et dans l'encyclopédie de chimie industrielle de Ullmann.

A titre d'exemples de matières colorantes minérales, on peut citer le dioxyde de titane, traité ou non traité en surface, l'oxyde de zinc, les oxydes de zirconium ou de cérium, les oxydes de fer ou de chrome, le violet de manganèse, le bleu outremer, l'hydrate de chrome et le bleu ferrique. Par exemple, les pigments minéraux suivants peuvent être utilisés : Ta₂O₅, Ti₃O₅, Ti₂O₃, TiO, ZrO₂ en mélange avec TiO₂, ZrO₂, Nb₂O₅, CeO₂, ZnS.

A titres d'exemples de matières colorantes organiques, on peut citer les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les matières colorantes peuvent être choisies parmi le carmin, le noir de carbone, le noir d'aniline, le jaune azo, la quinacridone, le bleu de phtalocyanine, le rouge sorgho, les pigments bleus codifiés dans le Color Index sous les références Cl 42090, 69800, 69825, 73000, 74100, 74160, les pigments jaunes codifiés dans le Color Index sous les références Cl 11680, 11710, 15985, 19140, 20040, 21100, 21108, 47000, 47005, les pigments verts codifiés dans le Color Index sous les références Cl 61565, 61570, 74260, les pigments oranges codifiés dans le Color Index sous les références Cl 11725, 15510, 45370, 71105, les pigments rouges codifiés dans le Color Index sous les références Cl 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Les matières colorantes peuvent être choisies parmi les colorants, les laques ou les pigments.

Les colorants sont par exemple des colorants liposolubles, bien que les colorants hydrosolubles puissent être utilisés. Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).

Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, la silice, le borosilicate de calcium et de sodium ou le borosilicate de calcium et d'aluminium, et l'aluminium. Parmi les colorants organiques, on peut citer le carmin de cochenille.

A titre d'exemples de laques, on peut citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

Par pigments, il faut comprendre des particules blanches ou colorées, minérales ou organiques, destinées à colorer et/ou opacifier la composition. Les pigments conformes à l'invention peuvent par exemple être choisis parmi les pigments blancs ou colorés, les pigments à effets spéciaux tels que les nacres, les pigments réfléchissants ou les pigments interférentiels.

Comme pigments utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0,001 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les pigments peuvent être présents dans la composition à raison de 0,05 à 30 % du poids de la composition finale, et de préférence à raison de 2 à 20 %.

La variété des pigments qui peuvent être utilisés dans la présente invention permet d'obtenir une riche palette de couleurs, ainsi que des effets optiques particuliers tels que des effets métalliques ou interférentiels.

Par pigments à effets spéciaux, on entend les pigments qui créent d'une manière générale une apparence colorée (caractérisé par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semi-transparente ou transparente classique.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre de pigments nacrés, on peut citer les nacres Cellini commercialisée par Engelhard (Mica-TiO2-laque), Prestige commercialisée par Eckart (Mica-TiO2), Colorona commercialisée par Merck (Mica-TiO2-Fe2O3).

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, et les pigments thermochromiques.

La composition contient avantageusement des pigments goniochromatiques, par exemple des pigments multicouches interférentiels, et/ou des pigments réfléchissants. Ces deux types de pigments sont décrits dans la demande FR0209246 dont le contenu est incorporé par référence dans la présente demande.

La composition peut contenir des pigments réfléchissants, qui peuvent être ou non goniochromatiques, interférentielles ou non.

Leur taille est compatible avec la manifestation d'une réflexion spéculaire de la lumière visible (400-700 nm), d'intensité suffisante, compte tenu de la brillance moyenne de la composition, pour créer un point de surbrillance. Cette taille est susceptible de varier selon la nature chimique des particules, leur forme et leur pouvoir de réflexion spéculaire de la lumière visible.

Les particules réfléchissantes présenteront de préférence une dimension d'au moins 10 µm, par exemple comprise entre environ 20 µm et environ 50 µm.

Par « dimension », on désigne la dimension donnée par la distribution granulométrique statistique à la moitié de la population, dite D50. La taille des particules réfléchissantes pourra dépendre de leur état de surface. Plus celui-ci est réfléchissant, plus la dimension pourra, a priori, être faible, et inversement.

Des particules réfléchissantes utilisables dans l'invention, à reflet métallique ou blanc, peuvent par exemple réfléchir la lumière dans toutes les composantes du visible sans absorber de manière significative une ou plusieurs longueurs d'ondes. La réflectance spectrale de ces particules réfléchissantes peut par exemple être supérieure à 70 % dans l'intervalle 400-700 nm, et mieux d'au moins 80 %, voire 90 % ou encore 95 %.

Les particules réfléchissantes quelque soit leur forme, peuvent présenter une structure multicouche ou non et, dans le cas d'une structure multicouche, par exemple au moins une couche d'épaisseur uniforme, notamment d'un matériau réfléchissant, qui enrobe un substrat.

Le substrat peut être choisi par les verres, les céramiques, le graphite, les oxydes métalliques, les alumines, les silices, les silicates, notamment les aluminosilicates et les borosilicates et le mica synthétique, cette liste n'étant pas limitative.

Le matériau réfléchissant peut comporter une couche de métal ou d'un composé métallique.

La couche de métal ou de composé métallique peut enrober ou non en totalité le substrat et la couche de métal peut être au moins partiellement recouverte par une couche d'un autre matériau, par exemple un matériau transparent. Il peut être préférable que la couche de métal ou de composé métallique enrobe en totalité, directement ou indirectement, c'est-à-dire avec interposition d'au moins une couche intermédiaire, métallique ou non, le substrat.

Le métal peut être choisi par exemple parmi Ag, Au, Cu, Al, Ni, Sn, Mg, Cr, Mo, Ti, Pt, Va, Rb, W, Zn, Ge, Te, Se et leurs alliages. Ag, Au, Al, Zn, Ni, Mo, Cr, Cu et leurs alliages (par exemple les bronzes et les laitons) sont des métaux préférés.

Dans le cas notamment de particules à substrat enrobé d'argent ou d'or, la couche métallique peut être présente à une teneur représentant par exemple de 0,1 à 50 % du poids total des particules, voire entre 1 et 20 %.

Des particules de verre recouvertes d'une couche métallique sont décrites notamment dans les documents JP-A-09188830, JP-A-10158450, JP-A-10158541, JP-A-07258460 et JP-A-05017710.

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Les particules réfléchissantes quelque soit leur forme, peuvent également être choisies parmi les particules à substrat synthétique enrobé au moins partiellement d'au moins une couche d'au moins un composé métallique, notamment un oxyde métallique, choisi par exemple parmi les oxydes de titane, notamment TiO₂, de fer notamment Fe₂O₃, d'étain, de chrome, le sulfate de baryum et les composés suivants : MgF₂, CrF₃, ZnS, ZnSe, SiO₂, Al₂O₃, MgO, Y₂O₃, SeO₃, SiO, HfO₂, ZrO₂, CeO₂, Nb₂O₅, Ta₂O₅, MoS₂ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, soit d'oxyde de titane, d'oxyde d'étain ou d'un de leurs mélanges comme celles vendues sous la marque REFLECKS^{®} par la société ENGELHARD.

Conviennent également à l'invention, les pigments de la gamme METASHINE 1080R commercialisée par la société NIPPON SHEET GLASS CO. LTD. Ces pigments, plus particulièrement décrits dans la demande de brevet JP 2001-11340, sont des paillettes de verre C-GLASS comprenant 65 à 72 % de SiO₂, recouvertes d'une couche d'oxyde de titane de type rutile (TiO₂). Ces paillettes de verre ont une épaisseur moyenne de 1 micron et une taille moyenne de 80 microns soit un rapport en taille moyenne/épaisseur moyenne de 80. Elles présentent des reflets bleus, verts, jaunes ou de teinte argent selon l'épaisseur de la couche de TiO₂.

On peut encore citer les particules de dimension comprise entre 80 et 100 µm, comportant un substrat de mica synthétique (fluorophlogopite) revêtu de dioxyde de titane représentant 12% du poids total de la particule, vendues sous la dénomination PROMINENCE par la société NIHON KOKEN.

Les particules réfléchissantes peuvent encore être choisies parmi les particules formées d'un empilement d'au moins deux couches à indices de réfraction différents. Ces couches peuvent être de nature polymérique ou métallique et notamment inclure au moins une couche polymérique. De telles particules sont notamment décrites dans WO 99/36477, US 6 299 979 et US 6 387 498. A titre illustratif des matériaux pouvant constituer les différentes couches de la structure multicouche, on peut citer, cette liste n'étant pas limitative: le naphthalate de polyéthylène (PEN) et ses isomères, les téréphthalates de polyalkylene, et des polyimides. Des particules réfléchissantes comportant un empilement d'au moins deux couches de polymères sont commercialisées par la société 3M sous la dénomination MIRROR GLITTER. Ces particules comportent des couches de 2,6-PEN et de polyméthacrylate de méthyle dans un rapport massique de 80/20. De telles particules sont décrites dans le brevet US 5 825 643.

La composition peut contenir un ou plusieurs pigments goniochromatiques.

L'agent de coloration goniochromatique peut être choisi par exemple parmi les structures multicouches interférentielles et les agents de coloration à cristaux liquides.

Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.

La structure multicouche peut présenter ou non, par rapport à une couche centrale, une symétrie au niveau de la nature chimique des couches empilées.

Des exemples de structures multicouche interférentielles symétriques utilisables dans sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination CHROMAFLAIR par la société FLEX ; MoS₂/SiO₂/Al/SiO₂/MoS₂; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃; TiO₂/SiO₂/TiO₂ et TiO₂/Al₂O₃/TiO₂, SnO/TiO₂/SiO₂/TiO₂/SnO; Fe₂O₃/SiO₂/Fe₂O₃; SnO/mica/TiO₂/SiO₂/TiO₂/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut encore utiliser des agents de coloration goniochromatiques à structure multicouche comprenant une alternance de couches polymériques par exemple du type naphtalate de polyéthylène et téréphtalate de polyéthylène. De tels agents sont notamment décrits dans WO-A-96/19347 et WO-A-99/36478.

On peut citer, à titre d'exemple de pigments à structure multicouche polymérique, ceux commercialisés par la société 3M sous la dénomination COLOR GLITTER.

Les agents de coloration à cristaux liquides comprennent par exemple des silicones ou des éthers de cellulose sur lesquels sont greffés des groupes mésomorphes.

Comme particules goniochromatiques à cristaux liquides, on peut utiliser par exemple celles vendues par la société CHENIX ainsi que celle commercialisées sous la dénomination HELICONE^{®} HC par la société WACKER.

Les compositions selon l'invention peuvent se présenter sous toute forme acceptable et usuelle pour une composition cosmétique.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Les compositions conformes à l'invention peuvent être utilisées pour le soin ou le maquillage des matières kératiniques telles que les cheveux, la peau, les cils, les sourcils, les ongles, les lèvres, le cuir chevelu et plus particulièrement pour le maquillage des lèvres, des cils et/ou du visage.

Elles peuvent donc se présenter sous la forme d'un produit de soin et/ou de maquillage de la peau du corps ou du visage, des lèvres, des cils, des sourcils, des cheveux, du cuir cheveu ou des ongles; d'un produit solaire ou autobronzant; d'un produit capillaire notamment de coloration, de conditionnement et/ou de soin des cheveux; elles se présentent avantageusement sous forme de mascara, de rouge à lèvres, de brillant à lèvres (gloss), de fard à joues ou à paupières, de fond de teint.

L'invention a encore pour objet l'utilisation de deux polycondensats différents tels que décrits ci dessus, notamment dans les proportions et la constitution chimique décrites ciavant pour le maquillage des lèvres afin d'améliorer la tenue de la couleur dans le temps.

L'invention a encore pour objet un procédé de traitement cosmétique des matières kératiniques, notamment de la peau du corps ou du visage, des lèvres, des ongles, des cheveux et/ou des cils, comprenant l'application sur lesdites matières d'une composition cosmétique telle que définie précédemment.

Ce procédé selon l'invention permet notamment le soin ou le maquillage des lèvres, par application d'une composition de rouge à lèvres ou de brillant à lèvres (gloss) selon l'invention.

L'invention concerne également l'utilisation des compositions décrites précédemment pour le maquillage des lèvres.

La présente invention a également pour objet un ensemble cosmétique comprenant :
- un récipient délimitant au moins un compartiment, ledit récipient étant fermé par un élément de fermeture ; et
- une composition telle que décrite précédemment disposée à l'intérieur dudit compartiment.

Le récipient peut être sous toute forme adéquate. Il peut être notamment sous forme d'un flacon, d'un tube, d'un pot, d'un étui, d'une boite, d'un sachet ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, ou un clapet.

Le récipient peut être associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être sous forme d'un pinceau, tel que décrit par exemple dans le brevet FR 2 722 380. L'applicateur peut être sous forme d'un bloc de mousse ou d'élastomère, d'un feutre, ou d'une spatule. L'applicateur peut être libre (houppette ou éponge) ou solidaire d'une tige portée par l'élément de fermeture, tel que décrit par exemple dans le brevet US 5 492 426. L'applicateur peut être solidaire du récipient, tel que décrit par exemple le brevet FR 2 761 959.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, serrage, soudage, collage, ou par attraction magnétique. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient. Alternativement, notamment lorsque le produit est sous forme d'un stick, ce dernier peut être entraîné par un mécanisme à piston. Toujours dans le cas d'un stick, notamment de produit de maquillage (rouge à lèvres, fond de teint, etc.), le récipient peut comporter un mécanisme, notamment à crémaillère, ou avec une tige filetée, ou avec une rampe hélicoïdale, et apte à déplacer un stick en direction de ladite ouverture. Un tel mécanisme est décrit par exemple dans le brevet FR 2 806 273 ou dans le brevet FR 2 775 566. Un tel mécanisme pour un produit liquide est décrit dans le brevet FR 2 727 609.

Le récipient peut être constitué d'un boîtier avec un fond délimitant au moins un logement contenant la composition, et un couvercle, notamment articulé sur le fond, et apte à recouvrir au moins en partie ledit fond. Un tel boîtier est décrit par exemple dans la demande WO 03/018423 ou dans le brevet FR 2 791 042.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

La composition peut être à la pression atmosphérique à l'intérieur du récipient (à température ambiante) ou pressurisée, notamment au moyen d'un gaz propulseur (aérosol). Dans ce dernier cas, le récipient est équipé d'une valve (du type de celles utilisées pour les aérosols).

L'invention est illustrée plus en détail dans les exemples suivants.

### Méthode de mesure de la viscosité

La viscosité à 80°C ou à 110°C du polymère est mesurée à l'aide d'un viscosimètre à cône plan de type BROOKFIELD CAP 1000+.

Le cône-plan adapté est déterminé par l'homme du métier, sur la base de ses connaissances; notamment :
- entre 50 et 500 mPa.s, on peut utiliser un cône 02
- entre 500 et 1000 mPa.s : cône 03
- entre 1000 et 4000 mPa.s : cône 05
- entre 4000 et 10000 mPa.s : cône 06

### Exemple 1 : Synthèse du pentaérythrityl benzoate/isophtalate/isostéarate

Dans un réacteur équipé d'une agitation mécanique, d'une arrivée d'argon et d'un système de distillation, on charge 20 g d'acide benzoïque, 280 g d'acide isostéarique et 100 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 11 heures. On refroidit à une température comprise entre 100 et 130°C puis on introduit 100 g d'acide isophtalique et on chauffe à nouveau progressivement jusqu'à 220°C pendant environ 11 heures.

On obtient ainsi 405 g de polycondensat pentaérythrityl benzoate/isophta-late/isostéarate sous la forme d'une huile très épaisse.

Le polycondensat présente les caractéristiques suivantes :
- soluble à 50% en poids, à 25°C, dans le Parléam
- Indice d'acide = 3,7
- Indice d'hydroxyle = 72
- Mw = 59400
- η_{110°C} = 1510 mPa.s
- rapport entre le nombre de mole d'acide monocarboxylique aromatique et le nombre de mole d'acide monocarboxylique ramifié non aromatique : 0,16.

### Exemple 2 : Synthèse du pentaérythrityl benzoate/isoahtalate/isostéarate

Dans un réacteur équipé d'une agitation mécanique, d'une arrivée d'argon et d'un système de distillation, on charge 35 g d'acide benzoïque, 270 g d'acide isostéarique et 80 g de pentaérythritol puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 11 heures. On refroidit à une température comprise entre 100 et 130°C puis on introduit 65 g d'acide isophtalique et on chauffe à nouveau progressivement jusqu'à 220°C pendant environ 5 heures.

On obtient ainsi 380 g de polycondensat pentaérythrityl benzoate/isophta-late/isostéarate sous la forme d'une huile.

Le polycondensat présente les caractéristiques suivantes :
- soluble à 50% en poids, à 25°C, dans le Parléam
- Indice d'acide = 5,5
- Indice d'hydroxyle = 103
- Mw = 7200
- η_{80°C} = 700 mPa.s
- rapport entre le nombre de mole d'acide monocarboxylique aromatique et le nombre de mole d'acide monocarboxylique ramifié non aromatique : 0,30.

### Exemple 3 : Synthèse du pentaérythrityl benzoate/isoahtalate/stéarate

Dans un réacteur équipé d'une agitation mécanique, d'une arrivée d'argon et d'un système de distillation, on charge 10 g d'acide benzoïque, 370 g d'acide stéarique et 95 g de pentaérythritol puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 11 heures. On refroidit à une température comprise entre 100 et 130°C puis on introduit 90 g d'acide isophtalique et on chauffe à nouveau progressivement jusqu'à 220°C pendant environ 11 heures.

On obtient ainsi 430 g de polycondensat pentaérythrityl benzoate/isophta-late/stéarate sous la forme d'une huile très épaisse.

Le polycondensat présente les caractéristiques suivantes :
- soluble à 50% en poids, à 70°C, dans le Parléam
- Indice d'acide = 10,8
- Mw = 8800
- η_{80°C} = 360 mPa.s

### Exemples A à R

De manière similaire aux exemples précédents, on prépare les polycondensats suivants (les % sont en poids):

| | Polyol (% et nature) | Acide aromatique (% et nature) | Acide polycarboxylique ou anhydride (% et nature) | Acide non aromatique (% et nature) | Solubilité * |
|---|---|---|---|---|---|
| Exemple A | 21,6 Pentaérythritol | 3,9 benzoïque | 19,5 Acide isophtalique | 27,5% isostéarique + 27,5% isononanoïque | à 25°C |
| Exemple B | 16,8 Pentaérythritol | 1,8 benzoïque | 15,9 Acide isophtalique | 65,5 béhénique | à 70°C |
| Exemple C | 20 Pentaérythritol | 4 terbutylbenzoïque | 20 Acide isophtalique | 56 isostéarique | à 25°C |
| Exemple D | 17,4 glycérol | 8,6 benzoïque | 16 acide isophtalique | 58 isostéarique | à 25°C |
| Exemple E | 20,7 glycérol | 8,5 terbutylbenzoique | 15,9 Acide adipique | 54,9 isononanoïque | à 25°C |
| Exemple F | 25,5 diglycérol | 2 benzoïque | 13,7 Acide isophtalique | 58,8 isononanoïque | à 25°C |
| Exemple G | 28 ditrimethylol-propane | 2 1-naphtoique | 14 acide isophtalique | 56 isostéarique | à 25°C |
| Exemple H | 25,2 trimethylol-propane | 5,8 benzoïque | 12,6 acide isophtalique | 56,3 isononanoïque | à 25°C |
| Exemple I | 25 trimethylol-propane | 2,1 m-toluique | 14,6 anhydride phtalique | 58,3 isostéarique | à 25°C |
| Exemple J | 21,9 érythritol | 6,3 terbutylbenzoique | 13,5 acide sébacique | 58,3 isooctanoïque | à 25°C |
| Exemple K | 20,4 dipentaérythritol | 6,1 benzoïque | 20,4 PRIPOL 1009** | 53,1 isostéarique | à 25°C |
| Exemple L | 28 ditrimethylol-propane | 2 1-naphtoique | 14 acide isophtalique | 40% isostéarique + 16% 2-ethylhexanoique | à 25°C |
| Exemple M | 21,3 pentaérythritol | 6,4 benzoïque | 17 Acide succinique | 27,7% nonanoïque + 27,6% isoheptanoïque | à 25°C |
| Exemple N | 17,4 glycérol | 8,6 benzoïque | 16 acide isophtalique | 58 stéarique | à 70°C |
| Exemple O | 25,5 diglycérol | 2 benzoïque | 13,7 Acide isophtalique | 58,8 myristique | à 70°C |
| Exemple P | 25,5 diglycérol | 3,9 benzoïque | 15,7 Acide sébacique | 54,9 laurique | à 70°C |
| Exemple Q | 20,4 dipentaérythritol | 6,1 benzoïque | 20,4 PRIPOL 1009** | 53,1 béhénique | à 70°C |
| Exemple R | 25,2 triméthylolpropane | 5,8 benzoïque | 12,6 Acide isophtalique | 31,1% stéarique + 25,3 % béhénique | à 70°C |

| | | | | | |
|---|---|---|---|---|---|
| * 'à 25°C' indique que le polymère est soluble à 50% en poids, à 25°C, dans le Parléam; 'à 70°C' indique que le polymère est soluble à 50% en poids, à 70°C, dans le Parléam ** PRIPOL 1009 d'Uniqema : dimère d'acide oléïque | | | | | |

### Exemple 4 : Synthèse du pentaérythrityl benzoate/isophtalate/isostéarate/ stéarate

Dans un réacteur équipé d'une agitation mécanique, d'une arrivée d'argon et d'un système de distillation, on charge 20 g d'acide benzoïque, 210 g d'acide stéarique, 70 g d'acide isostéarique et 100 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 11 heures. On refroidit à une température comprise entre 100 et 130°C puis on introduit 100 g d'acide isophtalique et on chauffe à nouveau progressivement jusqu'à 220°C pendant environ 11 heures.

On obtient ainsi 450 g de polycondensat pentaérythrityl benzoate/isophtalate/isostéarate /stéarate sous la forme d'une huile très épaisse.

Le polycondensat présente les caractéristiques suivantes :
- soluble à 50% en poids, à 70°C, dans le Parléam
- Indice d'acide = 7,1
- η_{110°C} = 850 mPa.s
- Mw = 28500
- rapport entre le nombre de mole d'acide monocarboxylique aromatique et le nombre de mole d'acides monocarboxyliques non aromatiques : 0,166.

### Exemple 5 : Synthèse du pentaérythrityl béhénate/benzoate/isophtalate /isostéarate

Dans un réacteur équipé d'une agitation mécanique, d'une arrivée d'argon et d'un système de distillation, on charge 20 g d'acide benzoïque, 140 g d'acide béhénique, 140 g d'acide isostéarique et 100 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 11 heures. On refroidit à une température comprise entre 100 et 130°C puis on introduit 100 g d'acide isophtalique et on chauffe à nouveau progressivement jusqu'à 220°C pendant environ 11 heures.

On obtient ainsi 440 g de polycondensat pentaérythrityl béhénate/benzoate/ isophtalate/isostéarate sous la forme d'une huile très épaisse.

Le polycondensat présente les caractéristiques suivantes :
- soluble à 50% en poids, à 70°C, dans le Parléam
- Indice d'acide = 4,2
- η_{110°C} = 2050 mPa.s
- rapport entre le nombre de mole d'acide monocarboxylique aromatique et le nombre de mole d'acides monocarboxyliques non aromatiques : 0,181.

### Exemples a à j

De manière similaire aux exemples précédents, on prépare les polycondensats suivants (les % sont en poids):

| | Polyol (% et nature) | Acide aromatique (% et nature) | Acide polycarboxylique ou anhydride (% et nature) | Acides non aromatiques (% et nature) | Solubilité * |
|---|---|---|---|---|---|
| Exemple a | 20,4 Pentaérythritol | 4,1 benzoïque | 18,3 Acide isophtalique | 28,6% isostéarique + 14,3% isononanoïque + 14,3% stéarique | à 25°C |
| Exemple b | 20 Pentaérythritol | 4 benzoïque | 20 Acide isophtalique | 18% isostéarique + 38% stéarique | à 25°C |
| Exemple c | 20 Pentaérythritol | 4 benzoïque | 20 Acide isophtalique | 28% isostéarique + 28% stéarique | à 25°C |
| Exemple d | 19,8 Pentaérythritol | 4 benzoïque | 19,8 Acide isophtalique | 40,6% isostéarique + 15,8% stéarique | à 25°C |
| Exemple e | 19,8 Pentaérythritol | 4 benzoïque | 19,8 Acide isophtalique | 48,5% isostéarique + 7,9% stéarique | à 25°C |
| Exemple f | 19,8 Pentaérythritol | 4 benzoïque | 19,8 Acide isophtalique | 52,4% isostéarique + 4% stéarique | à 25°C |
| Exemple g | 25,5 Diglycérol | 3,9 benzoïque | 15,7 Acide sébacique | 34,9% isostéarique + 20% laurique | à 25°C |
| Exemple h | 25 triméthylol-propane | 2,1 m-toluique | 14,6 anhydride phtalique | 18,3% isostéarique + 40% behenique | à 70°C |
| Exemple i | 21,9 érythritol | 6,3 terbutyl- benzoique | 13,5 Acide sébacique | 8,3% isooctanoïque + 50% stéarique | à 70°C |
| Exemple j | 20,7 glycérol | 8,5 terbutyl-benzoique | 15,9 acide adipique | 45,9% isononanoïque + 9% béhénique | à 25°C |

| | | | | | |
|---|---|---|---|---|---|
| * 'à 25°C' indique que le polymère est soluble à 50% en poids, à 25°C, dans le Parléam; 'à 70°C' indique que le polymère est soluble à 50% en poids, à 70°C, dans le Parléam. | | | | | |

### Exemple 6 : Synthèse du pentaérythrityl benzoate/isophtalate/laurate/PDMS

Dans un réacteur équipé d'une agitation mécanique, d'une arrivée d'argon et d'un système de distillation, on charge 150 g d'acide benzoïque, 165 g d'acide laurique et 110 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 15 heures. On refroidit à une température comprise entre 100 et 130°C puis on introduit 90 g d'acide isophtalique et 50 g de Silicone α,ω diol X22-160AS de Shin-Etsu, et on chauffe à nouveau progressivement jusqu'à 220°C pendant environ 11 heures.

On obtient ainsi 510 g de polycondensat pentaérythrityl benzoate/isophtalate/laurate /PDMS sous la forme d'une huile épaisse qui se solidifie à température ambiante.

Le polycondensat présente les caractéristiques suivantes :
- Indice d'acide = 28,7
- Indice d'hydroxyle = 85
- η_{110°C} = 2,1 Poises (soit 210 mPa.s)
- rapport entre le nombre de mole d'acide monocarboxylique aromatique et le nombre de mole d'acide monocarboxylique ramifié non aromatique : 1,49.

On prélève 500 g de polycondensat obtenu ci-dessus, on le chauffe à 70°C et on coule lentement 215 g d'acétate d'éthyle sous agitation puis on clarifie par filtration à chaud sur fritté n°2. On obtient après refroidissement à température ambiante 705 g de solution de polycondensat à 70% dans l'acétate d'éthyle se présentant sous la forme d'un liquide visqueux jaune pâle et possédant une viscosité à 25°C d'environ 165 centipoises (mPa.s).

### Exemple 7 : Synthèse du pentaérythrityl benzoate/isophtalate/laurate

Dans un réacteur équipé d'une agitation mécanique, d'une arrivée d'argon et d'un système de distillation, on charge 165 g d'acide benzoïque, 160 g d'acide laurique et 120 g de pentaérythritol puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 15 heures. On refroidit à une température comprise entre 100 et 130°C puis on introduit 100 g d'acide isophtalique et on chauffe à nouveau progressivement jusqu'à 220°C pendant environ 12 heures.

On obtient ainsi 510 g de polycondensat pentaérythrityl benzoate/isophtalate/laurate sous la forme d'une huile épaisse qui se solidifie à température ambiante.

Le polycondensat présente les caractéristiques suivantes :
- Indice d'acide = 20,4
- Indice d'hydroxyle = 66
- η_{110°C} = 4,7 Poises (soit 470 mPa.s)
- rapport entre le nombre de mole d'acide monocarboxylique aromatique et le nombre de mole d'acide monocarboxylique ramifié non aromatique : 1,69.

On prélève 500 g de polycondensat obtenu ci-dessus, le chauffe à 70°C et on coule lentement 215 g d'acétate d'éthyle sous agitation puis on clarifie par filtration à chaud sur fritté n°2. On obtient après refroidissement à température ambiante 700 g de solution de polycondensat à 70% dans l'acétate d'éthyle se présentant sous la forme d'un liquide visqueux jaune pâle et possédant une viscosité à 25°C d'environ 310 centipoises (mPa.s).

### Exemple 8 : Synthèse du pentaérythrityl benzoate/phtalate/laurate

Dans un réacteur équipé d'une agitation mécanique, d'une arrivée d'argon et d'un système de distillation, on charge 185 g d'acide benzoïque, 174 g d'acide laurique et 114,6 g de pentaérythritol puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 18 heures. On refroidit à une température comprise entre 100 et 130°C puis on introduit 80 g d'anhydride phtalique et on chauffe à nouveau progressivement jusqu'à 220°C pendant environ 8 heures. On ajoute 15 g de pentaérythritol et on maintient 8 heures à 220°C.

On obtient ainsi 512 g de polycondensat pentaérythrityl benzoate/phta-late/laurate sous la forme d'une huile épaisse qui se solidifie à température ambiante.

Le polycondensat présente les caractéristiques suivantes :
- Indice d'acide = 13,0
- Indice d'hydroxyle = 60
- η_{110°C} = 0,9 Poises (soit 90 mPa.s)
- rapport entre le nombre de mole d'acide monocarboxylique aromatique et le nombre de mole d'acide monocarboxylique ramifié non aromatique : 1,74.

### Exemple 9 de rouge à lèvres en stick :

| | Ingrédient (nom INCI) | % W |
|---|---|---|
| A | TRIMETHYL PENTAPHENYL TRISILOXANE | 57.55 |
| | POLYCONDENSAT DE l'exemple 1 | 16.00 |
| B | MICROCRYSTALLINE WAX | 4.55 |
| | BEESWAX | 1.95 |
| C | POLYCONDENSAT DE l'exemple 3 | 1.00 |
| | BIS-DIGLYCERYL POLYACYLADIPATE-2 | 12 |
| | | |
| | OXYDE DE TITANE RUTILE TRAITE D ALUMINE/SILICE/TRI-METHYOLPROPANE | 0,20 |
| | LAQUE D'ALUMINIUM DE BLEU BRILLANT FCF SUR ALUMINE | 0,20 |
| | OXYDES DE FER BRUN,JAUNE | 0,95 |
| | LAQUE D'ALUMINIUM DE TARTRAZINE SUR ALUMINE | 0,85 |
| | SEL DE CALCIUM DU ROUGE LITHOL B | 0,45 |
| E | MICA-OXYDE DE TITANE | 2,80 |
| | MICA-OXYDE DE TITANE | 1,00 |
| | MICA-OXYDE DE TITANE | 0,50 |
| | Total | 100,00 |

## Revendications

1. Composition cosmétique comprenant
- au moins un premier polyester susceptible d'être obtenu par réaction :
- d'au moins un polyol comprenant 3 à 6 groupes hydroxyles;
- d'au moins un acide monocarboxylique ramifié non aromatique;
- d'au moins un acide monocarboxylique aromatique, et
- d'au moins un acide polycarboxylique comprenant au moins 2 groupes carboxyliques COOH et/ou un anhydride cyclique d'un tel acide polycarboxylique,
- au moins un deuxième polyester susceptible d'être obtenu par réaction :
- d'au moins un polyol comprenant 3 à 6 groupes hydroxyles;
- d'au moins un acide monocarboxylique linéaire non aromatique;
- d'au moins un acide monocarboxylique aromatique, et
- d'au moins un acide polycarboxylique comprenant au moins 2 groupes carboxyliques COOH et/ou un anhydride cyclique d'un tel acide polycarboxylique.

2. Composition selon l'une des revendications précédentes, dans laquelle le polyol est choisi parmi le glycérol, le pentaérythritol, le diglycérol, le sorbitol et leurs mélanges; et encore mieux est du pentaérythritol.

3. Composition selon l'une des revendications précédentes, dans laquelle l'acide monocarboxylique ramifié non aromatique est choisi parmi l'acide 2-éthylhexanoïque, l'acide isooctanoïque, l'isoheptanoïque, l'acide isononanoïque, l'acide isostéarique, et leurs mélanges.

4. Composition selon l'une des revendications précédentes, dans laquelle l'acide monocarboxylique linéaire non aromatique est choisi parmi l'acide laurique, l'acide myristique l'acide nonanoïque, l'acide palmitique, l'acide stéarique, l'acide béhénique et leurs mélanges.

5. Composition selon l'une des revendications précédentes, dans laquelle l'acide monocarboxylique aromatique est choisi parmi l'acide benzoïque, l'acide 4-tert-butyl-benzoïque, l'acide o-toluique, l'acide m-toluique, l'acide 1-naphtoïque, seuls ou en mélanges; et encore mieux l'acide benzoïque seul.

6. Composition selon l'une des revendications précédentes, dans laquelle l'acide monocarboxylique aromatique, ou le mélange desdits acides, représente 0,5 à 9,95% en poids, mieux de 1 à 9,5% en poids, voire 1,5 à 8% en poids, du poids total du polyester final.

7. Composition selon l'une des revendications précédentes, dans laquelle l'acide polycarboxylique est choisi parmi les acides polycarboxyliques linéaires, ramifiés et/ou cycliques, saturés ou insaturés, voire aromatiques, comprenant 2 à 50, notamment 2 à 40, atomes de carbone, en particulier 3 à 36, voire 3 à 18, et encore mieux 4 à 12 atomes de carbone, voire 4 à 10 atomes de carbone; ledit acide comprenant au moins deux groupes carboxyliques COOH, de préférence de 2 à 4 groupes COOH.

8. Composition selon l'une des revendications précédentes, dans laquelle l'acide polycarboxylique ou son anhydride est choisi parmi l'acide adipique, l'anhydride phtalique et/ou l'acide isophtalique, et mieux l'acide isophtalique seul.

9. Composition selon l'une des revendications précédentes, dans laquelle l'acide polycarboxylique et/ou son anhydride cyclique représente 10 à 30% en poids, et mieux 14 à 25% en poids, du poids total du polyester.

10. Composition selon l'une des revendications précédentes, dans laquelle les polyesters sont susceptibles d'être obtenus par réaction :
- d'au moins un polyol choisi parmi, seul ou en mélange, le glycérol, le pentaérythritol, le sorbitol et leurs mélanges; présent en une quantité de 10 à 30% en poids, notamment 12 à 25% en poids, et mieux 14 à 22% en poids, par rapport au poids total du polyester final;
- d'au moins un acide monocarboxylique non aromatique;
présent en une quantité de 30 à 80% en poids, notamment 40 à 75% en poids, et mieux 45 à 70% en poids, par rapport au poids total du polyester final;
- d'au moins un acide monocarboxylique aromatique choisi parmi, seul ou en mélange, l'acide benzoïque, l'acide o-toluique, l'acide m-toluique, l'acide 1-naphtoïque; présent en une quantité de 0,1 à 10% en poids, notamment 1 à 9,5% en poids, voire 1,5 à 8% en poids par rapport au poids total du polyester final; et
- d'au moins un acide polycarboxylique ou un de ses anhydrides, choisi parmi, seul ou en mélange, l'anhydride phtalique et l'acide isophtalique; présent en une quantité de 5 à 40% en poids, notamment 10 à 30% en poids, et mieux 14 à 25% en poids, par rapport au poids total du polyester final.

11. Composition selon l'une des revendications précédentes, dans laquelle le premier polyester est présent en une quantité totale comprise entre 1 et 50% en poids, notamment entre 10 et 45% en poids, voire entre 10 et 20% en poids par rapport au poids de la composition.

12. Composition selon l'une des revendications précédentes, dans laquelle le deuxième polyester est présent en une quantité totale comprise entre 0,1 et 20% en poids, notamment entre 0,2 et 10% en poids, voire entre 0,5 et 2% en poids par rapport au poids de la composition

13. Composition cosmétique contenant :
- un premier polyester acide benzoïque / acide isophtalique / acide isostéarique / pentaérythritol, et
- un deuxième polyester acide benzoïque / acide isophtalique / acide stéarique / pentaérythritol.

14. Composition selon l'une des revendications précédentes, dans laquelle le premier polymère et le deuxième polymère sont dans un ratio massique compris entre 50/1 et 2/1, notamment de 30/1 à 20/1.
